# EUROPEAN PATENT APPLICATION

(11) **EP 4 557 302 A1**
(43) Date of publication of application: **21.05.2025**
(21) Application number: 24185885.1
(22) Date of filing: 02.07.2024
(51) Int. Cl.: G16H 10/20

(54) **METHODS AND SYSTEMS FOR AUTOMATED GENERATION OF CLINICAL TRIAL DOCUMENTS**

(30) Priority: 20.11.2023 IN 202321078787
(71) Applicant: Tata Consultancy Services Limited, Maharashtra (IN)
(72) Inventor: RAJBHOJ, ASHA SUSHILKUMAR, 411060 Pune, Maharashtra (IN); PATHAN, AJIM INNUS, 411057 Pune, Maharashtra (IN); NISTALA, PADMALATA VENKATA, 500034 Hyderabad, Telangana (IN); PANDEY, RAJESH, 400079 Mumbai, Maharashtra (IN); KULKARNI, VINAY, 411060 Pune, Maharashtra (IN)
(74) Representative: Goddar, Heinz J.

(57) **Abstract**

The disclosure relates generally to methods and systems for automated generation of clinical trial documents. Conventional technologies for automated clinical trial documents writing lack an end-to-end, efficient, and a holistic approach on automating the overall clinical trial documents writing process. Methods and systems of the present disclosure employ a clinical trial knowledge model that contains concepts, infotypes and contexts, a configurable dynamic recommendation model, and a clinical trial template model for generating the clinical trial documents. The present disclosure enables the digitalization of information from different sources of information using meta-model based approach. For a given clinical trial use case, the method of the present disclosure recommends the applicable concepts and infotypes. The recommendation provides a guided search of information, reduces search complexity, and finally generates formatted clinical trial documents.

## Description

### CROSS-REFERENCE TO RELATED APPLICATIONS AND PRIORITY

The present application claims priority to Indian application no. 202321078787, filed on November 20, 2023.

### TECHNICAL FIELD

The disclosure herein generally relates to document writing, and, more particularly, to methods and systems for automated generation of clinical trial documents.

### BACKGROUND

Pharmaceutical companies need to submit a huge volume of medical documents related to clinical trials, called as clinical trial documents, to drug regulatory authorities such as Food and Drug Administration (FDA) agency, to obtain approvals before marketing and releasing new medicines and drugs into the market. Multiple sources of information are to be referred to in order to write these clinical trial documents as per industry regulations and guidelines defined by the drug regulatory authority. Hence, it is important to accelerate clinical trials, make the clinical trial documents, and get necessary approvals from the drug regulatory authority, to bring new medicines, and drugs faster to the market to relieve patients from their suffering.

Typically, clinical trial documents are long documents, where each clinical trial document runs over 100 pages, is composed of many variables (typically above 150 variables) and needs to follow many document templates (protocols) defined by the drug regulatory authority. The manual writing process of the clinical trial documents is cumbersome, effort, time-consuming, and skill dependent as it involves searching the information strewn over multiple sources. Evolving regulations by the drug regulatory authority further impact the manual writing process of the clinical trial documents. Thus, creating the clinical trial documents is a time consuming process due to the information not being readily available and time lost in follow-ups with concerned teams to obtain the requisite information. Medical document writing concepts are composed of other multiple concepts or information types. As there is a lot of interdependence among such concepts and information types, a domain knowledge needs to be applied based on the clinical trial domain, specified diseases, therapy area, etc.

The knowledge of the clinical trial documents development exists in diverse document form, and that too is strewn over the multiple sources of the information. The fragmented and distributed nature of this information in natural language text makes this a herculean task. Low productivity of writing medical documents impacts the overall cycle of a drug approval process. The core clinical trial documents writing process is still manual. Hence, there is a need for guided clinical trial documents creation with less time and in an automated manner.

Conventional technologies for automated clinical trial documents writing are very limited. Conventional technologies for the automated clinical trial documents writing are mostly around text summarization techniques, lexeme hypotheses, and so on. Hence, the conventional technologies for the automated clinical trial documents writing lack an end-to-end, efficient, and a holistic approach on automating the overall clinical trial documents writing process.

### SUMMARY

Embodiments of the present disclosure present technological improvements as solutions to one or more of the above-mentioned technical problems recognized by the inventors in conventional systems.

In an aspect, a processor-implemented method for automated generation of clinical trial documents is provided. The method including the steps of: receiving, (i) one or more medical writing boilerplates associated with one or more disease types, (ii) one or more historical clinical trial documents, (iii) a plurality of clinical trial standard template documents defined for a drug regulatory authority, and (iv) a trial use case text to perform clinical trials for a drug; preprocessing, (i) the one or more medical writing boilerplates associated with the one or more disease types, and (ii) the one or more historical clinical trial documents, using one or more natural language processing (NLP) techniques, to obtain (i) one or more pre-processed medical writing boilerplates associated with the one or more disease types, and (ii) one or more pre-processed historical clinical trial documents; executing, one or more knowledge extraction patterns, on (i) the one or more pre-processed medical writing boilerplates associated with the one or more disease types, and (ii) the one or more pre-processed historical clinical trial documents, using a clinical trial knowledge meta model, to extract a clinical trial knowledge model, and wherein the clinical trial knowledge model comprises instances of (i) a plurality of medical writing (MW) concepts,(ii) a plurality of MW infotypes, (iii) a plurality of MW contexts, (iv) a plurality of MW groups, (v) one or more relations between each of the plurality of MW concepts, the plurality of MW infotypes, the plurality of MW contexts, and the plurality of MW groups, and (vi) one or more recommendation rules; preprocessing, the trial use case text, using the one or more natural language processing (NLP) techniques, the clinical trial knowledge model, and a domain dictionary, to identify one or more trial use case MW context parameters, and one or more trial use case MW infotypes, and to create a clinical trial instance of the trial use case text; recommending, (i) a plurality of relevant MW infotypes and (ii) a plurality of relevant MW concepts, using the one or more trial use case MW context parameters and the one or more trial use case MW infotypes identified from the trial use case text; generating, a clinical trial recommendation model, by attaching the plurality of relevant MW infotypes, the plurality of relevant MW concepts and the one or more trial use case MW context parameters, to the clinical trial instance, using a clinical trial recommendation meta model; executing, a template extraction pattern on the plurality of clinical trial standard template documents, using a clinical trial template meta model, to extract a clinical trial template model, wherein the clinical trial template model comprises a plurality of MW templates; generating, a plurality of clinical trial documents for the trial use case text, using the clinical trial recommendation model and the clinical trial template model; receiving, one or more recommendations from a user, based on the plurality of clinical trial documents generated for the trial use case text; and updating, the clinical trial knowledge model with the one or more recommendations.

In another aspect, a system for automated generation of clinical trial documents is provided. The system includes: a memory storing instructions; one or more Input/Output (I/O) interfaces; and one or more hardware processors coupled to the memory via the one or more I/O interfaces, wherein the one or more hardware processors are configured by the instructions to: receive (i) one or more medical writing boilerplates associated with one or more disease types, (ii) one or more historical clinical trial documents, (iii) a plurality of clinical trial standard template documents defined for a drug regulatory authority, and (iv) a trial use case text to perform clinical trials for a drug; preprocess (i) the one or more medical writing boilerplates associated with the one or more disease types, and (ii) the one or more historical clinical trial documents, using one or more natural language processing (NLP) techniques, to obtain (i) one or more pre-processed medical writing boilerplates associated with the one or more disease types, and (ii) one or more pre-processed historical clinical trial documents; execute one or more knowledge extraction patterns, on (i) the one or more pre-processed medical writing boilerplates associated with the one or more disease types, and (ii) the one or more pre-processed historical clinical trial documents, using a clinical trial knowledge meta model, to extract a clinical trial knowledge model, and wherein the clinical trial knowledge model comprises instances of (i) a plurality of medical writing (MW) concepts,(ii) a plurality of MW infotypes, (iii) a plurality of MW contexts, (iv) a plurality of MW groups, (v) one or more relations between each of the plurality of MW concepts, the plurality of MW infotypes, the plurality of MW contexts, and the plurality of MW groups, and (vi) one or more recommendation rules; preprocess the trial use case text, using the one or more natural language processing (NLP) techniques, the clinical trial knowledge model, and a domain dictionary, to identify one or more trial use case MW context parameters, and one or more trial use case MW infotypes, and to create a clinical trial instance of the trial use case text; recommend (i) a plurality of relevant MW infotypes and (ii) a plurality of relevant MW concepts, using the one or more trial use case MW context parameters and the one or more trial use case MW infotypes identified from the trial use case text; generate a clinical trial recommendation model, by attaching the plurality of relevant MW infotypes, the plurality of relevant MW concepts and the one or more trial use case MW context parameters, to the clinical trial instance, using a clinical trial recommendation meta model; execute a template extraction pattern on the plurality of clinical trial standard template documents, using a clinical trial template meta model, to extract a clinical trial template model, wherein the clinical trial template model comprises a plurality of MW templates; generate a plurality of clinical trial documents for the trial use case text, using the clinical trial recommendation model and the clinical trial template model; receive one or more recommendations from a user, based on the plurality of clinical trial documents generated for the trial use case text; and update the clinical trial knowledge model with the one or more recommendations.

In yet another aspect, there are provided one or more non-transitory machine-readable information storage mediums comprising one or more instructions which when executed by one or more hardware processors cause: receiving (i) one or more medical writing boilerplates associated with one or more disease types, (ii) one or more historical clinical trial documents, (iii) a plurality of clinical trial standard template documents defined for a drug regulatory authority, and (iv) a trial use case text to perform clinical trials for a drug; preprocessing (i) the one or more medical writing boilerplates associated with the one or more disease types, and (ii) the one or more historical clinical trial documents, using one or more natural language processing (NLP) techniques, to obtain (i) one or more pre-processed medical writing boilerplates associated with the one or more disease types, and (ii) one or more pre-processed historical clinical trial documents; executing one or more knowledge extraction patterns, on (i) the one or more pre-processed medical writing boilerplates associated with the one or more disease types, and (ii) the one or more pre-processed historical clinical trial documents, using a clinical trial knowledge meta model, to extract a clinical trial knowledge model, and wherein the clinical trial knowledge model comprises instances of (i) a plurality of medical writing (MW) concepts,(ii) a plurality of MW infotypes, (iii) a plurality of MW contexts, (iv) a plurality of MW groups, (v) one or more relations between each of the plurality of MW concepts, the plurality of MW infotypes, the plurality of MW contexts, and the plurality of MW groups, and (vi) one or more recommendation rules; preprocessing the trial use case text, using the one or more natural language processing (NLP) techniques, the clinical trial knowledge model, and a domain dictionary, to identify one or more trial use case MW context parameters, and one or more trial use case MW infotypes, and to create a clinical trial instance of the trial use case text; recommending (i) a plurality of relevant MW infotypes and (ii) a plurality of relevant MW concepts, using the one or more trial use case MW context parameters and the one or more trial use case MW infotypes identified from the trial use case text; generating a clinical trial recommendation model, by attaching the plurality of relevant MW infotypes, the plurality of relevant MW concepts and the one or more trial use case MW context parameters, to the clinical trial instance, using a clinical trial recommendation meta model; executing a template extraction pattern on the plurality of clinical trial standard template documents, using a clinical trial template meta model, to extract a clinical trial template model, wherein the clinical trial template model comprises a plurality of MW templates; generating a plurality of clinical trial documents for the trial use case text, using the clinical trial recommendation model and the clinical trial template model; receiving one or more recommendations from a user, based on the plurality of clinical trial documents generated for the trial use case text; and updating the clinical trial knowledge model with the one or more recommendations.

In an embodiment, the clinical trial knowledge meta model comprises the plurality of MW concepts, the plurality of MW contexts, the plurality of MW infotypes, the plurality of MW groups, one or more relations between each of the plurality of MW concepts, the plurality of MW infotypes, and the plurality of MW contexts, and wherein (i) one or more MW concepts of the plurality of MW concepts and one or more MW infotypes of the plurality of MW infotypes, are associated with a MW group of the plurality of MW groups, (ii) each of the plurality of MW concepts and each of the plurality of MW infotypes are associated with one or more MW contexts of the plurality of MW contexts, (iii) the one or more MW concepts of the plurality of MW concepts are associated with one or more inclusion and exclusion dependency relationships, (iv) the one or more MW infotypes of the plurality of MW infotypes are associated with the one or more inclusion and exclusion dependency relationships, (v) each of the plurality of MW concepts are associated with a MW infotype of the plurality of MW infotypes, (vi) each MW concept comprises a concept name and a concept description, (vii) each MW infotype comprises an infotype name, and (viii) the plurality of MW contexts comprises (a) a medical condition category, (b) a therapy area, and (c) a common category.

In an embodiment, preprocessing the trial use case text, using the one or more natural language processing (NLP) techniques, the clinical trial knowledge model, and the domain dictionary, to identify one or more trial use case MW context parameters, and one or more trial use case MW infotypes, and to create the clinical trial instance of the trial use case text, comprising: traversing a logical subtree of the trial use case text, to identify a stream of words comprising variants of noun, verb, adjective, and adverb, using the one or more NLP techniques; matching each word of the stream of words with the domain dictionary and the clinical trial knowledge model, using a matching algorithm, to identify the one or more trial use case MW context parameters, and the one or more trial use case MW infotypes; and creating the clinical trial instance of the trial use case text, by attaching the one or more trial use case MW context parameters and the one or more trial use case MW infotypes.

In an embodiment, recommending the plurality of relevant MW infotypes, using the one or more trial use case MW context parameters and the one or more trial use case MW infotypes identified from the trial use case text, comprising: identifying a first set of MW infotypes out of the plurality of MW infotypes present in the clinical trial knowledge model, based on relationships between the one or more trial use case MW context parameters of the trial use case text and the plurality of MW infotypes present in the clinical trial knowledge model; identifying a second set of MW infotypes out of the plurality of MW infotypes present in the clinical trial knowledge model by inferring the first set of MW infotypes with respect to the one or more recommendation rules of the clinical trial knowledge model; identifying a third set of MW infotypes out of the plurality of MW infotypes present in the clinical trial knowledge model using one or more inclusion and exclusion dependency relationships obtained for the first set of MW infotypes and the second set of MW infotypes; and combining the first set of MW infotypes, the second set of MW infotypes, and the third set of MW infotypes, to obtain the plurality of relevant MW infotypes.

In an embodiment, recommending the plurality of relevant MW concepts, using the one or more trial use case MW context parameters and one or more trial use case MW infotypes identified from the trial use case text, comprising: identifying a first set of MW concepts out of the plurality of MW concepts present in the clinical trial knowledge model, based on (i) relationships between the plurality of relevant MW infotypes and the plurality of MW concepts present in the clinical trial knowledge model, and (ii) relationships between the one or more trial use case MW context parameters and the plurality of MW concepts present in the clinical trial knowledge model; identifying a second set of MW concepts out of the plurality of MW concepts present in the clinical trial knowledge model by inferring the first set of MW concepts and the plurality of relevant MW infotypes with respect to the one or more recommendation rules of the clinical trial knowledge model; identifying a third set of MW concepts out of the plurality of MW concepts present in the clinical trial knowledge model, using one or more inclusion and exclusion dependency relationships obtained for the first set of MW concepts and the second set of MW concepts; and combining the first set of MW concepts, the second set of MW concepts, and the third set of MW concepts, to obtain the plurality of relevant MW concepts.

In an embodiment, generating the plurality of clinical trial documents for the trial use case text, using the clinical trial recommendation model and the clinical trial template model, comprising: creating a clinical trial document for each MW template in the clinical trial template model, wherein each MW template comprises a plurality of MW sections and one or more MW sub-sections in each of the plurality of MW sections, wherein each MW section comprises a section name and a section description, and each sub-section comprises a sub-section name and a sub-section description; matching the section name of each of the plurality of MW sections and the sub-section name of each of the one or more MW sub-sections, with the plurality of relevant MW infotypes and the plurality of relevant MW concepts using the one or more NLP techniques; generating a MW concept description for each of the plurality of relevant MW concepts, using the plurality of relevant MW infotypes; inserting the MW concept description for each of the plurality of MW sections and each of the one or more MW sub-sections whose section name, and sub-section name is matching with the plurality of relevant MW concepts; inserting a MW infotype name for each of the plurality of MW sections and each of the one or more MW sub-sections whose section name, and sub-section name is matching with the plurality of relevant MW infotypes; generating a section description for each of the plurality of MW sections and each of the one or more MW sub-sections whose section name, and sub-section name is not matching with the plurality of relevant MW concepts and the plurality of relevant MW infotypes, using the plurality of relevant MW infotypes; and saving the clinical trial document created for each MW template, to obtain the plurality of clinical trial documents for the trial use case text, from the plurality of MW templates.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is an exemplary block diagram of a system for automated generation of clinical trial documents, in accordance with some embodiments of the present disclosure.
FIG. 2 is an exemplary block diagram illustrating a plurality of modules of the system of FIG. 1, for automated generation of clinical trial documents, in accordance with some embodiments of the present disclosure.
FIGS. 3A-3B illustrate exemplary flow diagrams of a processor-implemented method for automated generation of clinical trial documents, using the system of FIG. 1, in accordance with some embodiments of the present disclosure.
FIGS. 4A-4C depict a block diagram of an exemplary clinical trial knowledge meta model, in accordance with some embodiments of the present disclosure.
FIG. 5 is a block diagram of an exemplary rule meta model of the exemplary clinical trial knowledge meta model of FIGS. 4A-4C, in accordance with some embodiments of the present disclosure.
FIG. 6 is a flowchart comprising steps for preprocessing the trial use case text, using the one or more natural language processing (NLP) techniques, the clinical trial knowledge model, and the domain dictionary, to identify the one or more trial use case MW context parameters, and the one or more trial use case MW infotypes, and to create the clinical trial instance of the trial use case text, in accordance with some embodiments of the present disclosure.
FIG. 7 is a flowchart comprising steps for recommending the plurality of relevant MW infotypes, using the one or more trial use case MW context parameters and the one or more trial use case MW infotypes identified from the trial use case text, in accordance with some embodiments of the present disclosure.
FIG. 8 is a flowchart comprising steps for recommending the plurality of relevant MW concepts, from the one or more trial use case MW context parameters and one or more trial use case MW infotypes identified from the trial use case text, in accordance with some embodiments of the present disclosure.
FIG. 9 is a block diagram of an exemplary clinical trial recommendation meta model, in accordance with some embodiments of the present disclosure.
FIG. 10 is a block diagram of an exemplary clinical trial template meta model, in accordance with some embodiments of the present disclosure.
FIG. 11 is a flowchart comprising steps for generating the plurality of clinical trial documents for the trial use case text, using the clinical trial recommendation model and the clinical trial template model, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Development and introduction of a new drug or a new medicine takes about 6-10 years, before going into the market. Research studies reveal that 25% of the above-mentioned time on an average is dedicated to a development of different types of scientific or operational documents typically called as medical documents or clinical trial documents required for submission to drug regulatory authorities such as food and drug administration (FDA) agency for an approval.

There are limited conventional techniques in the art for the automated clinical trial documents writing however, they do not clearly propose a holistic solution to digitalize a variety of concepts related to medical document writing especially clinical trial documents. Further, the conventional techniques are mostly around text summarization techniques, lexeme hypotheses, and so on. None of the conventional techniques support automated trial use case analysis, trial context parameters based intelligent search, creation of reusable trial model that can be used across multiple documents to be created for each trial and composing medical writing documents in a harmonized and integrated manner.

The present disclosure solves the technical problems in the art with the methods and systems for automated generation of clinical trial documents. According to the present disclosure, the methods and systems employ a clinical trial knowledge model that contains concepts, infotypes and contexts, a configurable dynamic recommendation model, and a clinical trial template model for generating the clinical trial documents.

The present disclosure enables the digitalization of information from different sources of information using meta-model based approach. For a given clinical trial use case text, the method of the present disclosure recommends the applicable concepts and information types. The recommendation provides a guided search of information, reduces search complexity, and finally generates a formatted clinical trial documents.

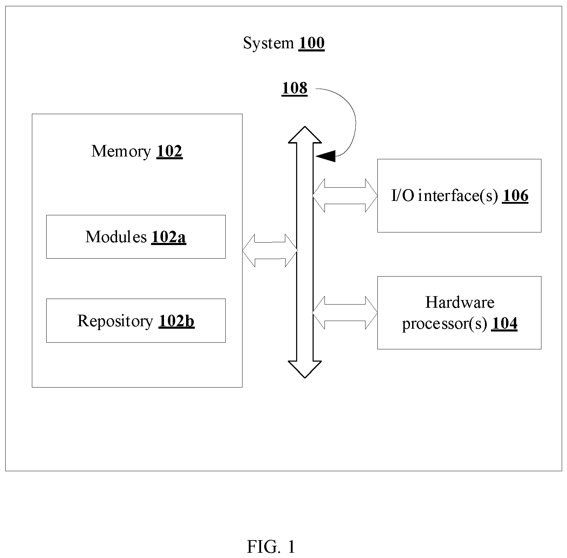

Referring now to the drawings, and more particularly to FIG. 1 through FIG. 11, where similar reference characters denote corresponding features consistently throughout the figures, there are shown preferred embodiments, and these embodiments are described in the context of the following exemplary system and/or method.

FIG. 1 is an exemplary block diagram of a system 100 for automated generation of clinical trial documents, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer, and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and `hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks, or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

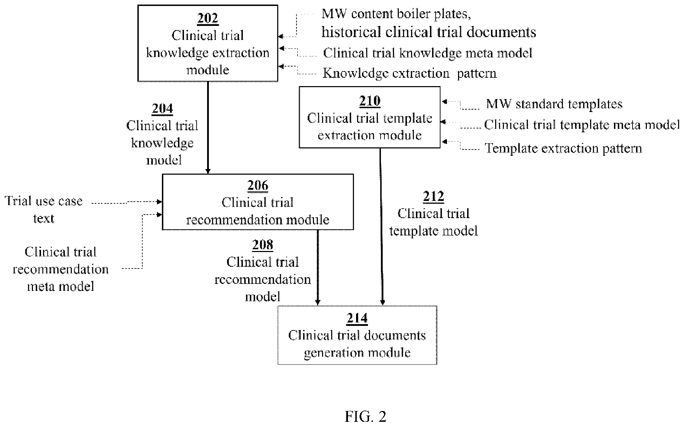

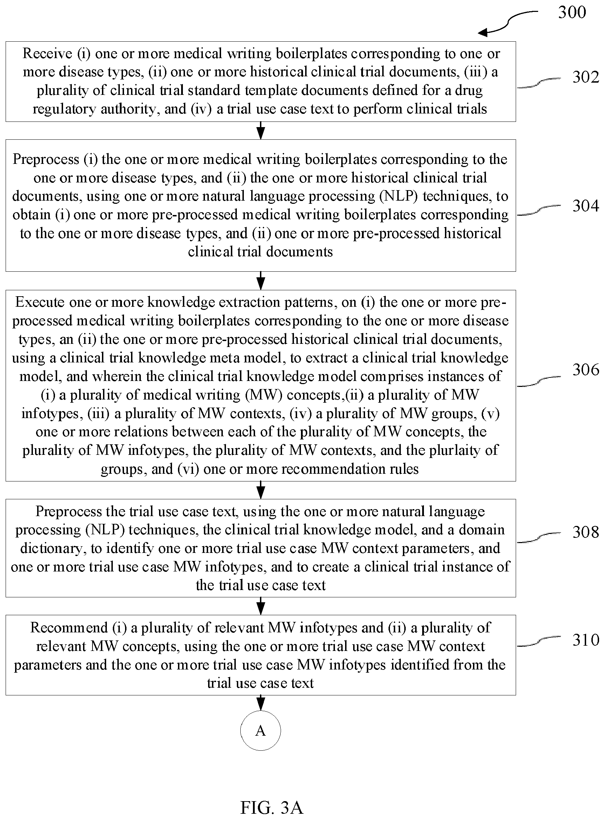

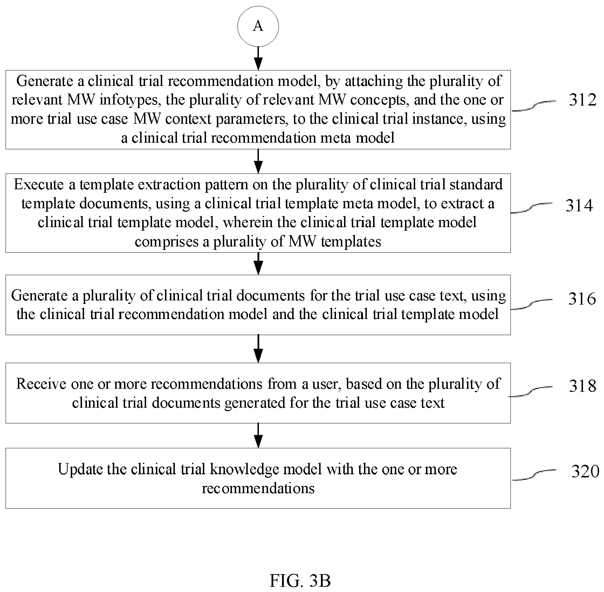

Referring collectively to FIG. 2 and FIGS. 3A-3B, components and functionalities of the system 100 are described in accordance with an example embodiment of the present disclosure. For example, FIG. 2 is an exemplary block diagram illustrating a plurality of modules 102a of the system 100 of FIG. 1, for automated generation of clinical trial documents, in accordance with some embodiments of the present disclosure. In an embodiment, the plurality of modules 102a include a clinical trial knowledge extraction module 202, a clinical trial knowledge model 204, a clinical trial recommendation module 206, a clinical trial recommendation model 208, a clinical trial template extraction module 210, a clinical trial template model 212, and a clinical trial documents generation module 214.

For example, FIGS. 3A-3B illustrate exemplary flow diagrams of a processor-implemented method 300 for automated generation of clinical trial documents, using the system 100 of FIG. 1, in accordance with some embodiments of the present disclosure. Although steps of the method 300 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 302 of the method 300, the one or more hardware processors 104 of the system 100 are configured to receive, (i) one or more medical writing boilerplates, and (ii) one or more historical clinical trial documents, (iii) a plurality of clinical trial standard template documents, and (iv) a trial use case text.

In an embodiment, the one or more medical writing boilerplates include the common clinical trial information source documents associated with one or more disease types, and disease specific information sources related documents. In an embodiment, the one or more historical clinical trial documents are the clinical trial documents that are already submitted in the past. In an embodiment, the plurality of clinical trial standard template documents are the standard template documents defined for a drug regulatory authority such as a food and drug administration (FDA) agency. In an embodiment, the trial use case text defines the trial use case objective for which the clinical trials are performed for a given drug or a medicine.

At step 304 of the method 300, the one or more hardware processors 104 of the system 100 are configured to preprocess the one or more medical writing boilerplates and the one or more historical clinical trial documents received at step 302 of the method, to obtain one or more pre-processed medical writing boilerplates associated with the one or more disease types, and one or more pre-processed historical clinical trial documents, respectively. In an embodiment, one or more natural language processing (NLP) techniques are employed in the preprocessing. In an embodiment, the preprocessing includes but are not limited to structurization, normalization, and standardization the one or more medical writing boilerplates and the one or more historical clinical trial documents, to obtain the one or more pre-processed medical writing boilerplates, and the one or more pre-processed historical clinical trial documents, respectively. In an embodiment, the structurization includes but is not limited to formatting, templatization based on a defined structure, and arranging in the defined structure. In an embodiment, the normalization includes but is not limited to removing extra spaces, removing irrelevant information such as page numbers, and line numbers, and filling in missing information. In an embodiment, the standardization includes but is not limited to arranging in defined standards.

At step 306 of the method 300, the one or more hardware processors 104 of the system 100 are configured to execute one or more knowledge extraction patterns, on the (i) one or more pre-processed medical writing boilerplates associated with the one or more disease types, (ii) one or more pre-processed historical clinical trial documents obtained at step 304 of the method 300, using a clinical trial knowledge meta model, to extract the clinical trial knowledge model 202.

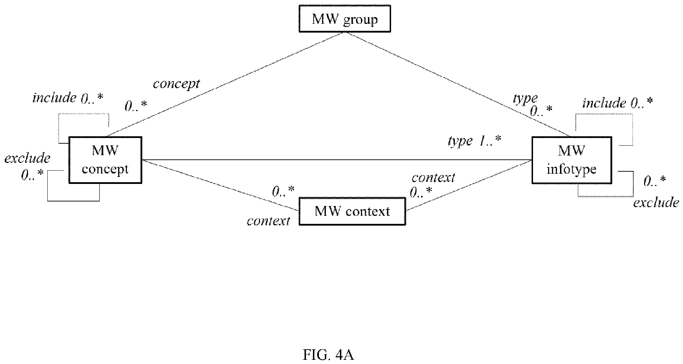

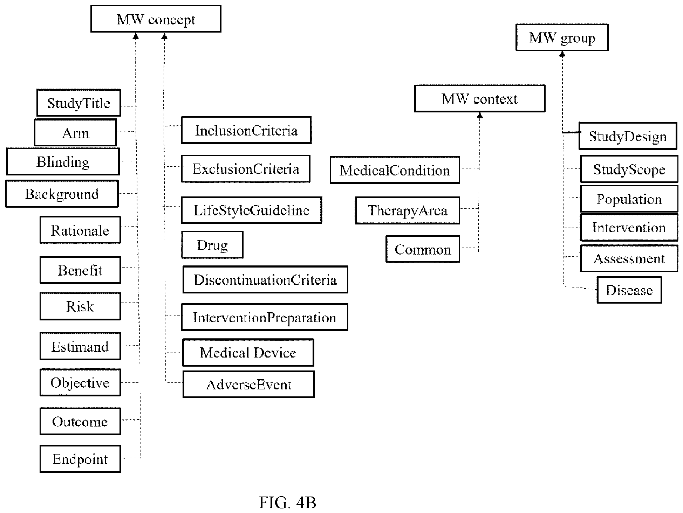

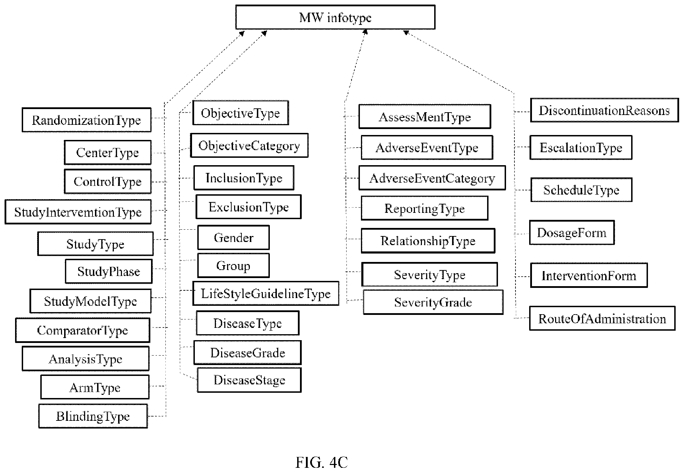

FIGS. 4A-4C depict a block diagram of an exemplary clinical trial knowledge meta model, in accordance with some embodiments of the present disclosure. As shown in FIGS. 4A-4C, the clinical trial knowledge meta model helps in defining a plurality of medical writing related concepts (MW concepts), a plurality of medical writing related information types (MW infotypes), a plurality of medical writing related context parameters (MW contexts), and one or more relations between each of the plurality of MW concepts, the plurality of MW infotypes, and the plurality of MW contexts. The related MW concepts and the MW infotypes are grouped through a MW group of a plurality of medical writing related groups (MW groups). Each MW concept has a concept name and a concept description. This is specified in information type property that can save binary data. Similarly, each MW infotype includes an infotype name. Each MW concept and each MW infotype may be applicable to a specific MW context. This is specified by association concept and type respectively with the MW context.

Further, the MW context helps in classifying the MW concept and the MW infotype into broadly three types - a medical condition, a therapy area, and common category. The common category type indicates a common knowledge applicable to all medical trials irrespective of the medical condition and the therapy area.

Examples of common MW Info type 'StudyType' are:
*Diagnostic*
*Therapeutic*
*Prevention*
*Early deduction*/ *screening*
*Treatment*
*Genetic Trials*
*Quality of life*
*Supportive care*
*Observational*
*Prospective*
Examples of common MW Info type 'Group' are:
*Healthy individuals*
*Patients*
*Gender specific*
*Disease specific*
*Geo specific*
*Adults*
*Pediatric*
*Geriatric*

Example of common MW concept 'StudyTitle' is*:defaultTitle: "A(n), [StudyPhase], [RandomizationType], [BlindingType], [ControlType], [CenterType], [ComparatorType], [StudyType], [ArmType] study to investigate [Outcome] with [Drug] [InterventionForm] compared with [Drug] [InterventionForm] in participants [Group] with [MedicalCondition] "*
wherein brackets of concept text indicate references of MW concept (Drug, Outcome), MW infotype (StudyPhase, RandomizationType, BlindingType, ControlType, CenterType, ComparatorType, StudyType, ArmType, InterventionForm), and MW context (MedicalCondition).

Other MW concepts and the MW infotypes could be specific to the medical condition and the therapy area. The MW infotypes may be referred to MW concept text, which is specified by type association. From the core abstract classes MW concepts and MW infotypes, multiple specific classes are inherited. For example, the inherited classes from the MW concepts are StudyTitle, Background, Rational, Benefit, Risk, Estimand, and so on. For example, the inherited classes from MW infotypes are RandomisationType, CenterType, ObjectiveType, and so on.

In an embodiment, the one or more relations include one or more inclusion and exclusion dependency relationships of the one or more MW concepts of the plurality of MW concepts, and the one or more inclusion and exclusion dependency relationships of the one or more MW infotypes of the plurality of MW infotypes.

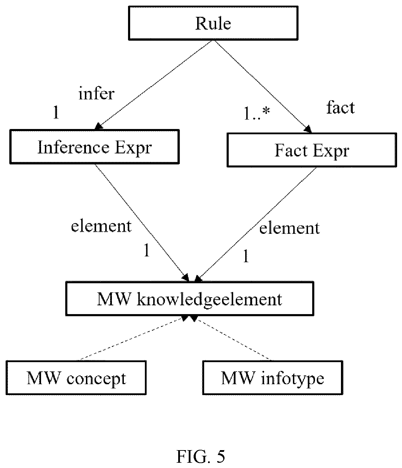

Further, the clinical trial knowledge meta model also includes a rule meta model. FIG. 5 is a block diagram of an exemplary rule meta model of the exemplary clinical trial knowledge meta model of FIGS. 4A-4C, in accordance with some embodiments of the present disclosure. As shown in FIG. 5, the exemplary rule meta model includes the rules for the MW infotypes and the MW concepts of the exemplary clinical trial knowledge meta model. Each rule has a Fact expression (Fact Expr) and Inference Expression (Inference Expr). The MW infotype / MW concept is inferred based on relevant instances associated with the MW infotype / MW concept of the Fact Expr.

In an embodiment, the clinical trial knowledge extraction module 202 configured to extract the clinical trial knowledge model 204 by instantiating the clinical trial knowledge meta model. More specifically, the clinical trial knowledge model 204 the includes instances of (i) the plurality of medical writing (MW) concepts, (ii) the plurality of MW infotypes, (iii) the plurality of MW contexts, (iv) the one or more relations between each of the plurality of MW concepts, the plurality of MW infotypes, and the plurality of MW contexts, of the clinical trial knowledge meta model. These instances are captured based on the one or more pre-processed medical writing boilerplates associated with the one or more disease types, and the one or more pre-processed historical clinical trial documents. The clinical trial knowledge model 204 also includes the one or more recommendation rules that are defined using the rule meta model, like the same way the clinical trial knowledge meta model has the rule meta model. Furthermore, the one or more recommendation rules defined by the rule model, are captured based on the one or more pre-processed medical writing boilerplates associated with the one or more disease types, and the one or more pre-processed historical clinical trial documents.

At step 308 of the method 300, the one or more hardware processors 104 of the system 100 are configured to preprocess the trial use case text received at step 302 of the method 300, to identify one or more trial use case MW context parameters, and one or more trial use case MW infotypes. Again, the one or more natural language processing (NLP) techniques are employed along with the clinical trial knowledge model 204 extracted at step 306 of the method 300, and a domain dictionary, to identify the one or more trial use case MW context parameters, and the one or more trial use case MW infotypes.

The one or more trial use case MW context parameters are the MW contexts discussed in the above steps but are present in the trial use case text. Similarly, the one or more trial use case MW infotypes are the MW infotypes discussed in the above steps but are present in the trial use case text. The one or more trial use case MW context parameters, and the one or more trial use case MW infotypes, identified at this step 308 are used to create a clinical trial instance of the trial use case text.

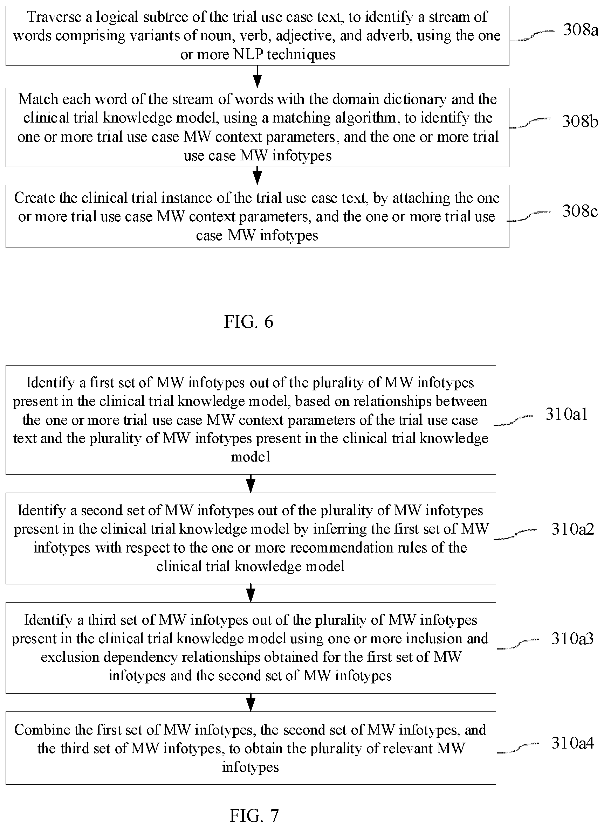

FIG. 6 is a flowchart comprising steps for preprocessing the trial use case text, using the one or more natural language processing (NLP) techniques, the clinical trial knowledge model 204, and the domain dictionary, to identify the one or more trial use case MW context parameters, and the one or more trial use case MW infotypes, and to create the clinical trial instance of the trial use case text, in accordance with some embodiments of the present disclosure. As shown in FIG. 6, identifying the one or more trial use case MW context parameters, and the one or more trial use case MW infotypes, and to creating the clinical trial instance of the trial use case text, is further explained through steps 308a through 308c.

At step 308a, the words present in the trial use case text are arranged in a logical subtree using the one or more NLP techniques, to identify a stream of words comprising variants of noun, verb, adjective, and adverb.

Then at step 308b, each word of the stream of words identified at step 308a, are matched with the domain dictionary and the clinical trial knowledge model 204, using a matching algorithm, to identify the one or more trial use case MW context parameters, and the one or more trial use case MW infotypes. Finally at step 308c, the clinical trial instance of the trial use case text, is created by attaching the one or more trial use case MW context parameters, and the one or more trial use case MW infotypes obtained at step 308b.

At step 310 of the method 300, the one or more hardware processors 104 of the system 100 are configured to recommend, (i) a plurality of relevant MW infotypes, and (ii) a plurality of relevant MW concepts, by matching with the one or more trial use case MW context parameters and the one or more trial use case MW infotypes identified from the trial use case text at step 308 of the method 300.

In an embodiment, the plurality of relevant MW infotypes is the relevant MW infotypes out of the plurality of MW infotypes in the clinical trial knowledge model 204, for the trial use case text. Similarly, the plurality of relevant MW concepts are the relevant MW concepts out of the plurality of medical writing (MW) concepts in the clinical trial knowledge model 204, for the trial use case text.

FIG. 7 is a flowchart comprising steps for recommending the plurality of relevant MW infotypes, using the one or more trial use case MW context parameters and the one or more trial use case MW infotypes identified from the trial use case text, in accordance with some embodiments of the present disclosure. As shown in FIG. 7, recommending the plurality of relevant MW infotypes, using the one or more trial use case MW context parameters and the one or more trial use case MW infotypes identified from the trial use case text, is explained through steps 310a1 to 310a4.

At step 310a1, a first set of MW infotypes out of the plurality of MW infotypes present in the clinical trial knowledge model 204, is identified. The first set of MW infotypes includes one or more MW infotypes. The first set of MW infotypes is identified based on one or more relationships between the one or more trial use case MW context parameters of the trial use case text identified at step 308 of the method 300 and the plurality of MW infotypes present in the clinical trial knowledge model 204.

At step 310a2, a second set of MW infotypes is identified out of the plurality of MW infotypes present in the clinical trial knowledge model 204. The second set of MW infotypes includes the one or more MW infotypes. The second set of MW infotypes is identified by inferring the one or more MW infotypes in the first set of MW infotypes, with respect to the one or more recommendation rules of the clinical trial knowledge model 204.

At step 310a3, a third set of MW infotypes is identified, out of the plurality of MW infotypes present in the clinical trial knowledge model 204. The third set of MW infotypes includes the one or more MW infotypes. The third set of MW infotypes is identified using the one or more inclusion and exclusion dependency relationships obtained for the first set of MW infotypes and the second set of MW infotypes. Some of the one or more MW infotypes present in the first set of MW infotypes, the second set of MW infotypes, the third set of MW infotypes, may be common.

At step 310a4, the first set of MW infotypes, the second set of MW infotypes, and the third set of MW infotypes, are combined to obtain the plurality of relevant MW infotypes. After the combining process, the duplicate relevant MW infotypes are eliminated.

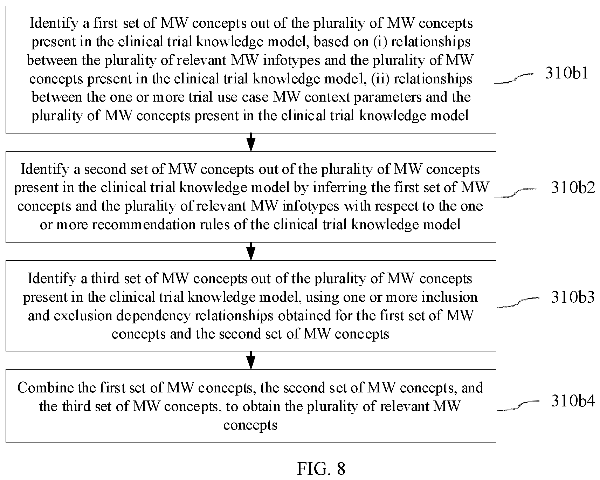

FIG. 8 is a flowchart comprising steps for recommending the plurality of relevant MW concepts, using the one or more trial use case MW context parameters and one or more trial use case MW infotypes identified from the trial use case text, in accordance with some embodiments of the present disclosure. As shown in FIG. 8, recommending the plurality of relevant MW concepts, using the one or more trial use case MW context parameters and the plurality of relevant MW infotypes identified in step 310a4, is explained through steps 310b1 to 310b4.

At step 310b1, a first set of MW concepts is identified out of the plurality of MW concepts present in the clinical trial knowledge model 204. The first set of MW concepts comprises one or more MW concepts. The first set of MW concepts is identified, based on (i) relationships between the plurality of relevant MW infotypes obtained at step 310a4, and the plurality of MW concepts present in the clinical trial knowledge model 204, and (ii) the relationships between the one or more trial use case MW context parameters and the plurality of MW concepts present in the clinical trial knowledge model 204.

At step 310b2, a second set of MW concepts is identified out of the plurality of MW concepts present in the clinical trial knowledge model 204. The second set of MW concepts comprises one or more MW concepts. The second set of MW concepts is identified by inferring the first set of MW concepts and the plurality of relevant MW infotypes with respect to the one or more recommendation rules of the clinical trial knowledge model 204.

At step 310b3, a third set of MW concepts is identified out of the plurality of MW concepts present in the clinical trial knowledge model 204. The third set of MW concepts comprises one or more MW concepts. The third set of MW concepts is identified based on the one or more inclusion and exclusion dependency relationships obtained for the first set of MW concepts identified at step 310b1 and the second set of MW concepts identified at step 310b2.

At step 310b4, the first set of MW concepts identified at step 310b1, the second set of MW concepts identified at step 310b2, and the third set of MW concepts identified at step 31 0b3, are combined to obtain the plurality of relevant MW concepts. After the combining process, the duplicate relevant MW concepts are eliminated.

At step 312 of the method 300, the one or more hardware processors 104 of the system 100 are configured to generate the clinical trial recommendation model 208, by attaching the plurality of relevant MW infotypes, the plurality of relevant MW concepts recommended at step 310 of the method 300, and the one or more trial use case MW context parameters obtained at step 308 of the method 300, to the clinical trial instance. In an embodiment, the clinical trial recommendation module 206 is configured to generate the clinical trial recommendation model 208, from a clinical trial recommendation meta model.

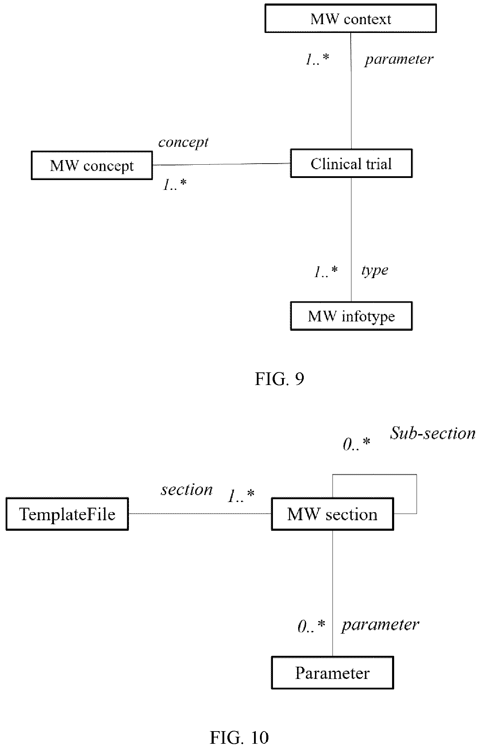

FIG. 9 is a block diagram of an exemplary clinical trial recommendation meta model, in accordance with some embodiments of the present disclosure. As shown in FIG. 9, the clinical trial recommendation meta model contains a plurality of recommendations about the plurality of relevant MW infotypes, the plurality of relevant MW concepts, and the one or more trial use case MW context parameters.

At step 314 of the method 300, the one or more hardware processors 104 of the system 100 are configured to execute a template extraction pattern on the plurality of clinical trial standard template documents received at step 302 of the method 300, to extract the clinical trial template model 212. A clinical trial template meta model is employed in this step to extract the clinical trial template model 212. In an embodiment, the clinical trial template extraction module 210 is configured to extract the clinical trial template model 212 from the plurality of clinical trial standard template documents. FIG. 10 is a block diagram of an exemplary clinical trial template meta model, in accordance with some embodiments of the present disclosure. In an embodiment, the clinical trial template model 212 comprises a plurality of MW templates.

At step 316 of the method 300, the one or more hardware processors 104 of the system 100 are configured to generate a plurality of clinical trial documents for the trial use case text, using the clinical trial recommendation model 208 and the clinical trial template model 212. More specifically, the plurality of MW templates of the clinical trial template model 212 is filled with the recommendations provided by the clinical trial recommendation model 208 to generate a plurality of clinical trial documents for the trial use case text. In an embodiment, the clinical trial documents generation module 214 is configured to generate the plurality of clinical trial documents for the trial use case text, using the clinical trial recommendation model 208 and the clinical trial template model 212.

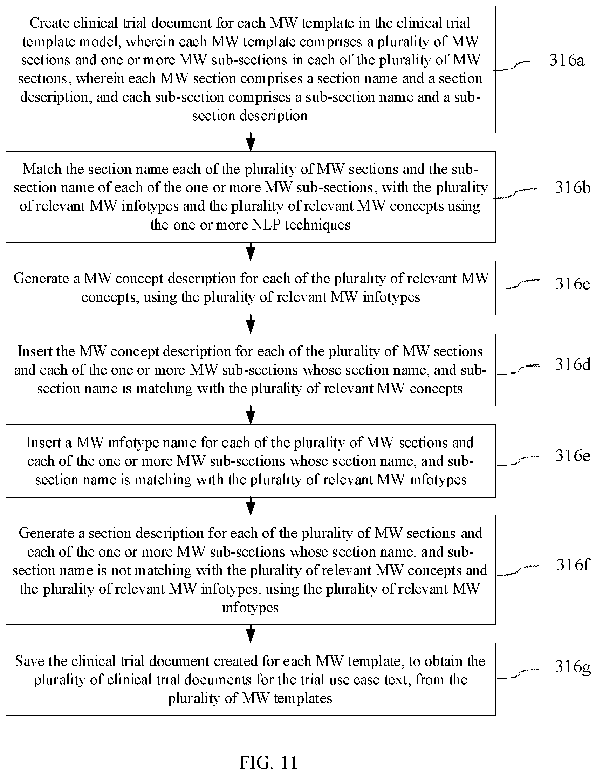

FIG. 11 is a flowchart comprising steps for generating the plurality of clinical trial documents for the trial use case text, using the clinical trial recommendation model 208 and the clinical trial template model 212, in accordance with some embodiments of the present disclosure. As shown in FIG. 11, generating the plurality of clinical trial documents for the trial use case text, using the clinical trial recommendation model 208 and the clinical trial template model 212, is explained through steps 316a to 316g.

At step 316a, a clinical trial document for each MW template of the plurality of MW templates in the clinical trial template model 212 is created. Each MW template comprises a plurality of MW sections and one or more MW sub-sections in each of the plurality of MW sections. Further, each MW section comprises a section name and a section description, and each sub-section comprises a sub-section name and a sub-section description. The style of the sections and sub-sections of each of the template documents are replicated in the corresponding MW sections and the sub-sections.

At step 316b, the name each of the plurality of MW sections and the name of each of the one or more MW sub-sections, are matched with the plurality of relevant MW infotypes and the plurality of relevant MW concepts from the clinical trial recommendation model 204 using the one or more NLP techniques.

At step 316c, a description for each of the plurality of relevant MW concepts, is generated using the plurality of relevant MW infotypes from the clinical trial recommendation model 208.

At step 316d, the MW concept description generated at step 316c, is inserted for each of the plurality of MW sections and each of the one or more MW sub-sections whose names are matching with the plurality of relevant MW concepts at step 316b.

At step 316e, a MW infotype name is inserted for each of the plurality of MW sections and each of the one or more MW sub-sections, whose names are matching with the plurality of relevant MW infotypes at step 316b.

At step 316f, a section description is generated for each of the plurality of MW sections and each of the one or more MW sub-sections, whose names are not matching with the plurality of relevant MW concepts and the plurality of relevant MW infotypes at step 316b. The section description is generated using the plurality of relevant MW infotypes.

At step 316g, the clinical trial document created for each MW template, is saved with the information inserted or generated through steps 316c through 316f, to obtain the plurality of clinical trial documents for the trial use case text, from the plurality of MW templates. The generated plurality of clinical trial documents is for the trial use case text received at step 302 of the method 300.

At step 318 of the method 300, the one or more hardware processors 104 of the system 100 are configured to receive one or more recommendations from a user, based on the plurality of clinical trial documents generated for the trial use case text. These recommendations are basically the feedback or the errors that are to be rectified in the plurality of clinical trial documents generated for the trial use case text at step 316 of the method 300.

At step 320 of the method 300, the one or more hardware processors 104 of the system 100 are configured to update the clinical trial knowledge model 204 with the one or more recommendations received at step 318 of the method 300. The updated clinical trial knowledge model 204 is then able to rectify the errors resulted in the past so that these errors are not repeated in future of the plurality of clinical trial documents for the given trial use case text.

The methods and systems of the present disclosure enable the digitalization of information from different sources of information using meta-model based approach. For a given clinical trial use case text, the present disclosure recommends the applicable MW concepts and the MW infotypes. The recommendation provides a guided search of information, reduces search complexity, and finally generates a formatted document. Below are some of the technical outcomes of the present disclosure:
- From quality perspective, the present disclosure reduces errors arising due to manual human errors owing to people's expertise gaps in understanding of problem domain concepts.
- From productivity perspective, the present disclosure reduces overall time in documents creation by improving accessibility to information and automated document creation.
- The present disclosure enables the reuse of trial information across different document creation.

The present disclosure invention provides jump-start for creation of clinical trial documents for a trial use case text through the automatic generation of formatted documents with all the information composed section-wise, that can be edited further as desired. This helps to achieve consistency, quality, and productivity in generating the clinical trial documents through significant technology enablement. Further, the present disclosure overcome the limitations of the conventional techniques, by auto generating documents for a given trial use case in a harmonized and integrated manner through intelligent combination of modeling technologies, NLP, search, rule engine, and document technologies.
- Harmonization of intelligent technologies for draft clinical trial document generation.
- Considering and provisioning for clinical trial use case context through automated analysis of the trial use case text.
- Domain dictionaries are defined and integrated to provide flexibility in matching.
- Generation of clinical trial document based on composition rules and template-based algorithm. A configurable clinical trial knowledge model provides flexibility and scalability to the system across medical writing domains and processes.

### Example scenario:

The medical writing boilerplates related to common MW infotypes applicable to all medical writing, and the historical clinical trial documents related to breast cancer and diabetes disease are considered to generate the clinical trial knowledge model. The knowledge extraction patterns were executed using a conventional `DocToModel' technique. Common protocol template (CPT) and clinical study report (CSR) standard templates were considered which are defined by a biopharma incorporation, and template loading extraction pattern executed using the conventional 'DocToModel' technique to generate the clinical trial template model.

Table 1 shows the details of the MW infotypes instantiated from the medical writing boilerplates and the historical clinical trial documents, using the clinical trial knowledge meta model to extract the clinical trial knowledge model.

**Table 1**

| MW infotypes | Total |
|---|---|
| AdverseEventCategory | 6 |
| AdverseEventType | 12 |
| AnalysisType | 6 |
| ArmType | 5 |
| AssessmentType | 20 |
| BlindingType | 7 |
| CenterType | 2 |
| ComparatorType | 5 |
| ControlType | 2 |
| DiscontinuationReasons | 26 |
| DiseaseGrade | 3 |
| DiseaseStage | 8 |
| DiseaseType | 13 |
| DosageForm | 6 |
| EscalationType | 2 |
| ExclusionType | 6 |
| Gender | 3 |
| Group | 9 |
| InclusionType | 9 |
| LifeStyleGuidelineType | 3 |
| Obj ectiveCategory | 12 |
| ObjectiveType | 7 |
| RandomizationType | 2 |
| RelationshipType | 5 |
| ReportingType | 2 |
| RouteOfAdministration | 7 |
| ScheduleType | 3 |
| SeverityGrade | 5 |
| SeverityType | 5 |
| StudyInterventionType | 5 |
| StudyModelType | 2 |
| StudyPhase | 10 |
| StudyType | 10 |

Table 2 shows the details of the MW concepts instantiated from the medical writing boilerplates and the historical clinical trial documents, using the clinical trial knowledge meta model to extract the clinical trial knowledge model.

**Table 2**

| MW concept | Medical Condition-Breast Cancer | Medical Condition-Diabetes | Total |
|---|---|---|---|
| Background | 2 | 0 | 2 |
| DiscontinuationCriteria | 1 | 0 | 1 |
| Endpoint | 28 | 14 | 42 |
| ExclusionCriteria | 12 | 19 | 31 |
| InclusionCriteria | 19 | 6 | 25 |
| Objective | 23 | 10 | 33 |

Table 3 shows the number of MW sections, and the parameters present in the exemplary templates CPT and the CSR:

**Table 3**

| | CPT | CSR |
|---|---|---|
| MW Sections | 42 | 99 |
| Parameters | 40 | 77 |

Below is the exemplary clinical trial use case text considered for generating the clinical trial documents.
Input trial use case text: A prospective randomised, open, multicentre, phase III study to assess different Durations of Anastrozole therapy after 2 to 3 years Tamoxifen as Adjuvant therapy in postmenopausal women with breast cancer.
Extracted trial context parameters from the above exemplary clinical trial use case text are:
*Medical Condition: Breast Cancer*
*TherapyArea: Oncology*

Extracted MW infotypes from the above exemplary clinical trial use case text are:
*Study Type: Prospective*
*Study Phase: Phase III*
*Randomization Type: Randomized*
*Blinding Type: Open-label*
*Center Type: Multi-center*
*Gender: Female*

The plurality of relevant MW infotypes were identified as below:
First Set: MW infotype identification based on extracted trial context parameter
   - Disease Type *- HER Positive, HER Negative, HER2, ER Positive, ER Negative, PR Positive, PR Negative, ER* & *PR, Triple Negative*
   - StudyModelType - *interventional,*
   - Disease Grade - *Grade 1 (well differentiated), Grade 2 (moderately differentiated), Grade 3 (poorly differentiated)*
   - Disease Stage - *Stage 0, Stage 1, Stage 2, Stage 3, Stage 4, Stage 5, Early, Metastatic*
Second Set: MW infotypes identification based on the recommendation rules
   - *None*
Third Set: Dependency based identification of instances *StudyType* due to inclusion dependency relationship with *StudyModelType* from the First Set
   *Studytype: Diagnostic, Early deduction*/*screening, Treatment, Genetic Trials*

The plurality of relevant MW concepts were identified as below:
∘ First Set: MW concept identification based on the extracted trial context parameters
   ▪ EndPoint - *'Disease free survival (DFS)', 'Progression free survival(PFS)'*
   ▪ DiscontinuationCriteria: *Breast Cancer Discontinuation Criteria*
   ▪ InclusionCriteria:
      - >=*18 years,*
      - *Female,*
      - *Obtained written informed consent before entering the study,*
      - *Patients currently using adjuvant tamoxifen for a duration of 2 to 3 years after surgery of the primary breast tumor,*
      - *Postmenopausal at the time of randomization, according to one or more of the following:*
         - *aged 55 or more and natural amenorrhoea;*
         - *bilateral oophorectomy, irrespective of age;*
         - *Aged 45-54 years and FSH and 17 beta oestradiol values within the postmenopausal range confirmed by local laboratory values within the last three months (including: patients who have had a hysterectomy),*
      - *No distant metastasis at the time of randomization*
      - *No recurrences after the primary diagnosis and treatment of breast cancer*
   ▪ ExclusionCriteria:
      - *Performance status: Karnofski score 60% or less,*
      - *Patients who, for whatever reason (e.g., confusion, infirmity, alcoholism), are unlikely to comply with trial requirements,*
      - *Patients unwilling to stop taking any drug known to affect sex hormonal status (including HRT), or in whom it would be inappropriate to stop*
o Second Set: MW concept identification based on the recommendation rules of clinical trial knowledge model
   ▪ Objective
      - *To evaluate Disease free survival (DFS),*
      - *To evaluate Progression free survival (PFS),*
      - *Clinical Benefit - Assessment of Toxicity*
∘ Third Set: MW concept identification based on the inclusion and exclusion dependency relations
   ▪ ExclusionCriteria:
      - < *18 years,*
      - *Treatment with a non-approved or experimental drug during the 3 months before informed consent.*

Based on the plurality of relevant MW infotypes and the plurality of relevant MW concepts identified, the clinical trial recommendation model was generated. Using the clinical trial knowledge model and the clinical trial template model that are extracted, and the clinical trial recommendation model generated, 2 clinical trial documents (each for the CPT template and the CSR template) for the given exemplary clinical trial use case text are generated.

Further, the 2 clinical trial documents generated for the given exemplary clinical trial use case text are validated to calculate the accuracy of the present disclosure. The accuracy of the present disclosure is calculated based on the number of the generated sections with accurate text divided by the number of the template sections targeted for generation. Table 4 shows the validation results:

**Table 4**

| Trial | Template sections | Template sections targeted for generation | Generated sections with accurate text | Generated sections with inaccurate / incomplete text | Accuracy |
|---|---|---|---|---|---|
| CPT | 42 | 30 | 27 | 3 | 90.00% |
| CSR | 99 | 38 | 34 | 4 | 89.47% |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems of automated generation of clinical trial documents using the trial use case text. The present disclosure enables the digitalization of information from different sources of information using meta-model based approach. For a given clinical trial use case, the method of the present disclosure recommends the applicable MW concepts and MW infotypes. The recommendation provides a guided search of information, reduces search complexity, and finally generates a formatted document.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300), comprising:
receiving, via one or more hardware processors, (i) one or more medical writing boilerplates associated with one or more disease types, (ii) one or more historical clinical trial documents, (iii) a plurality of clinical trial standard template documents defined for a drug regulatory authority, and (iv) a trial use case text to perform clinical trials for a drug (302);
preprocessing, via the one or more hardware processors, (i) the one or more medical writing boilerplates associated with the one or more disease types, and (ii) the one or more historical clinical trial documents, using one or more natural language processing (NLP) techniques, to obtain (i) one or more pre-processed medical writing boilerplates associated with the one or more disease types, and (ii) one or more pre-processed historical clinical trial documents (304);
executing, via the one or more hardware processors, one or more knowledge extraction patterns, on (i) the one or more pre-processed medical writing boilerplates associated with the one or more disease types, and (ii) the one or more pre-processed historical clinical trial documents, using a clinical trial knowledge meta model, to extract a clinical trial knowledge model, and wherein the clinical trial knowledge model comprises instances of (i) a plurality of medical writing (MW) concepts, (ii) a plurality of MW infotypes, (iii) a plurality of MW contexts, (iv) a plurality of MW groups, (v) one or more relations between each of the plurality of MW concepts, the plurality of MW infotypes, the plurality of MW contexts, and the plurality of MW groups, and (vi) one or more recommendation rules (306);
preprocessing, via the one or more hardware processors, the trial use case text, using the one or more natural language processing (NLP) techniques, the clinical trial knowledge model, and a domain dictionary, to identify one or more trial use case MW context parameters and one or more trial use case MW infotypes, and to create a clinical trial instance of the trial use case text (308);
recommending, via the one or more hardware processors, (i) a plurality of relevant MW infotypes and (ii) a plurality of relevant MW concepts, using the one or more trial use case MW context parameters and the one or more trial use case MW infotypes identified from the trial use case text (310);
generating, via the one or more hardware processors, a clinical trial recommendation model, by attaching the plurality of relevant MW infotypes, the plurality of relevant MW concepts and the one or more trial use case MW context parameters, to the clinical trial instance, using a clinical trial recommendation meta model (312);
executing, via the one or more hardware processors, a template extraction pattern on the plurality of clinical trial standard template documents, using a clinical trial template meta model, to extract a clinical trial template model, wherein the clinical trial template model comprises a plurality of MW templates (314); and
generating, via the one or more hardware processors, a plurality of clinical trial documents for the trial use case text, using the clinical trial recommendation model and the clinical trial template model (316).

2. The processor implemented method as claimed in claim 1, wherein the clinical trial knowledge meta model comprises the plurality of MW concepts, the plurality of MW contexts, the plurality of MW infotypes, the plurality of MW groups, one or more relations between each of the plurality of MW concepts, the plurality of MW infotypes, and the plurality of MW contexts, and wherein:
(i) one or more MW concepts of the plurality of MW concepts and one or more MW infotypes of the plurality of MW infotypes, are associated with a MW group of the plurality of MW groups,
(ii) each of the plurality of MW concepts and each of the plurality of MW infotypes are associated with one or more MW contexts of the plurality of MW contexts,
(iii) the one or more MW concepts of the plurality of MW concepts are associated with one or more inclusion and exclusion dependency relationships,
(iv) the one or more MW infotypes of the plurality of MW infotypes are associated with the one or more inclusion and exclusion dependency relationships,
(v) each of the plurality of MW concepts are associated with a MW infotype of the plurality of MW infotypes,
(vi) each MW concept comprises a concept name and a concept description,
(vii) each MW infotype comprises an infotype name, and
(viii) the plurality of MW contexts comprises (a) a medical condition category, (b) a therapy area, and (c) a common category.

3. The processor implemented method as claimed in claim 1, wherein preprocessing the trial use case text, using the one or more natural language processing (NLP) techniques, the clinical trial knowledge model, and the domain dictionary, to identify one or more trial use case MW context parameters, and one or more trial use case MW infotypes, and to create the clinical trial instance of the trial use case text, comprising:
traversing a logical subtree of the trial use case text, to identify a stream of words comprising variants of noun, verb, adjective, and adverb, using the one or more NLP techniques (308a);
matching each word of the stream of words with the domain dictionary and the clinical trial knowledge model, using a matching algorithm, to identify the one or more trial use case MW context parameters and the one or more trial use case MW infotypes (308b); and
creating the clinical trial instance of the trial use case text, by attaching the one or more trial use case MW context parameters, and the one or more trial use case MW infotypes (308c).

4. The processor implemented method as claimed in claim 1, wherein recommending the plurality of relevant MW infotypes, using the one or more trial use case MW context parameters and the one or more trial use case MW infotypes identified from the trial use case text, comprising:
identifying a first set of MW infotypes out of the plurality of MW infotypes present in the clinical trial knowledge model, based on relationships between the one or more trial use case MW context parameters of the trial use case text and the plurality of MW infotypes present in the clinical trial knowledge model (310a1);
identifying a second set of MW infotypes out of the plurality of MW infotypes present in the clinical trial knowledge model by inferring the first set of MW infotypes with respect to the one or more recommendation rules of the clinical trial knowledge model (310a2);
identifying a third set of MW infotypes out of the plurality of MW infotypes present in the clinical trial knowledge model using one or more inclusion and exclusion dependency relationships obtained for the first set of MW infotypes and the second set of MW infotypes (310a3); and
combining the first set of MW infotypes, the second set of MW infotypes, and the third set of MW infotypes, to obtain the plurality of relevant MW infotypes (310a4).

5. The processor implemented method as claimed in claim 1, wherein recommending the plurality of relevant MW concepts, using the one or more trial use case MW context parameters and one or more trial use case MW infotypes identified from the trial use case text, comprising:
identifying a first set of MW concepts out of the plurality of MW concepts present in the clinical trial knowledge model, based on (i) relationships between the plurality of relevant MW infotypes and the plurality of MW concepts present in the clinical trial knowledge model, and (ii) relationships between the one or more trial use case MW context parameters and the plurality of MW concepts present in the clinical trial knowledge model (310b1);
identifying a second set of MW concepts out of the plurality of MW concepts present in the clinical trial knowledge model by inferring the first set of MW concepts and the plurality of relevant MW infotypes with respect to the one or more recommendation rules of the clinical trial knowledge model (310b2);
identifying a third set of MW concepts out of the plurality of MW concepts present in the clinical trial knowledge model, using one or more inclusion and exclusion dependency relationships obtained for the first set of MW concepts and the second set of MW concepts (310b3); and
combining the first set of MW concepts, the second set of MW concepts, and the third set of MW concepts, to obtain the plurality of relevant MW concepts (310b4).

6. The processor implemented method as claimed in claim 1, wherein generating the plurality of clinical trial documents for the trial use case text, using the clinical trial recommendation model and the clinical trial template model, comprising:
creating a clinical trial document for each MW template in the clinical trial template model, wherein each MW template comprises a plurality of MW sections and one or more MW sub-sections in each of the plurality of MW sections, wherein each MW section comprises a section name and a section description, and each sub-section comprises a sub-section name and a sub-section description (316a);
matching the section name of each of the plurality of MW sections and the sub-section name of each of the one or more MW sub-sections, with the plurality of relevant MW infotypes and the plurality of relevant MW concepts using the one or more NLP techniques (316b);
generating a MW concept description for each of the plurality of relevant MW concepts, using the plurality of relevant MW infotypes (316c);
inserting the MW concept description for each of the plurality of MW sections and each of the one or more MW sub-sections whose section name, and sub-section name is matching with the plurality of relevant MW concepts (316d);
inserting a MW infotype name for each of the plurality of MW sections and each of the one or more MW sub-sections whose section name, and sub-section name is matching with the plurality of relevant MW infotypes (316e);
generating a section description for each of the plurality of MW sections and each of the one or more MW sub-sections whose section name, and sub-section name is not matching with the plurality of relevant MW concepts and the plurality of relevant MW infotypes, using the plurality of relevant MW infotypes (316f); and
saving the clinical trial document created for each MW template, to obtain the plurality of clinical trial documents for the trial use case text, from the plurality of MW templates (316g).

7. The processor implemented method as claimed in claim 1, further comprising:
receiving, via the one or more hardware processors, one or more recommendations from a user, based on the plurality of clinical trial documents generated for the trial use case text (318); and
updating, via the one or more hardware processors, the clinical trial knowledge model with the one or more recommendations (320).

8. A system (100) comprising:
a memory (102) storing instructions;
one or more input/output (I/O) interfaces (106); and
one or more hardware processors (104) coupled to the memory (102) via the one or more I/O interfaces (106), wherein the one or more hardware processors (104) are configured by the instructions to:
receive (i) one or more medical writing boilerplates associated with one or more disease types, (ii) one or more historical clinical trial documents, (iii) a plurality of clinical trial standard template documents defined for a drug regulatory authority, and (iv) a trial use case text to perform clinical trials for a drug;
preprocess (i) the one or more medical writing boilerplates associated with the one or more disease types, and (ii) the one or more historical clinical trial documents, using one or more natural language processing (NLP) techniques, to obtain (i) one or more pre-processed medical writing boilerplates associated with the one or more disease types, and (ii) one or more pre-processed historical clinical trial documents;
execute one or more knowledge extraction patterns, on (i) the one or more pre-processed medical writing boilerplates associated with the one or more disease types, and (ii) the one or more pre-processed historical clinical trial documents, using a clinical trial knowledge meta model, to extract a clinical trial knowledge model, and wherein the clinical trial knowledge model comprises instances of (i) a plurality of medical writing (MW) concepts,(ii) a plurality of MW infotypes, (iii) a plurality of MW contexts, (iv) a plurality of MW groups, (iv) one or more relations between each of the plurality of MW concepts, the plurality of MW infotypes, the plurality of MW contexts, and the plurality of MW groups, and (v) one or more recommendation rules;
preprocess the trial use case text, using the one or more natural language processing (NLP) techniques, the clinical trial knowledge model, and a domain dictionary, to identify one or more trial use case MW context parameters and one or more trial use case MW infotypes, and to create a clinical trial instance of the trial use case text;
recommend (i) a plurality of relevant MW infotypes and (ii) a plurality of relevant MW concepts, using the one or more trial use case MW context parameters and the one or more trial use case MW infotypes identified from the trial use case text;
generate a clinical trial recommendation model, by attaching the plurality of relevant MW infotypes, the plurality of relevant MW concepts and the one or more trial use case MW context parameters, to the clinical trial instance, using a clinical trial recommendation meta model;
execute a template extraction pattern on the plurality of clinical trial standard template documents, using a clinical trial template meta model, to extract a clinical trial template model, wherein the clinical trial template model comprises a plurality of MW templates; and
generate a plurality of clinical trial documents for the trial use case text, using the clinical trial recommendation model and the clinical trial template model.

9. The system as claimed in claim 8, wherein the clinical trial knowledge meta model comprises the plurality of MW concepts, the plurality of MW contexts, the plurality of MW infotypes, the plurality of MW groups, one or more relations between each of the plurality of MW concepts, the plurality of MW infotypes, and the plurality of MW contexts, and wherein:
(i) one or more MW concepts of the plurality of MW concepts and one or more MW infotypes of the plurality of MW infotypes, are associated with a MW group of the plurality of MW groups,
(ii) each of the plurality of MW concepts and each of the plurality of MW infotypes are associated with one or more MW contexts of the plurality of MW contexts,
(iii) the one or more MW concepts of the plurality of MW concepts are associated with one or more inclusion and exclusion dependency relationships,
(iv) the one or more MW infotypes of the plurality of MW infotypes are associated with the one or more inclusion and exclusion dependency relationships,
(v) each of the plurality of MW concepts are associated with a MW infotype of the plurality of MW infotypes,
(vi) each MW concept comprises a concept name and a concept description,
(vii) each MW infotype comprises an infotype name, and
(viii) the plurality of MW contexts comprises (a) a medical condition category, (b) a therapy area, and (c) a common category.

10. The system as claimed in claim 8, wherein the one or more hardware processors (104) are configured to preprocess the trial use case text, using the one or more natural language processing (NLP) techniques, the clinical trial knowledge model, and the domain dictionary, to identify one or more trial use case MW context parameters, and one or more trial use case MW infotypes, and to create the clinical trial instance of the trial use case text, by:
traversing a logical subtree of the trial use case text, to identify a stream of words comprising variants of noun, verb, adjective, and adverb, using the one or more NLP techniques;
matching each word of the stream of words with the domain dictionary and the clinical trial knowledge model, using a matching algorithm, to identify the one or more trial use case MW context parameters, and the one or more trial use case MW infotypes; and
creating the clinical trial instance of the trial use case text, by attaching the one or more trial use case MW context parameters, and the one or more trial use case MW infotypes.

11. The system as claimed in claim 8, wherein the one or more hardware processors (104) are configured to recommend the plurality of relevant MW infotypes, using the one or more trial use case MW context parameters and the one or more trial use case MW infotypes identified from the trial use case text, by:
identifying a first set of MW infotypes out of the plurality of MW infotypes present in the clinical trial knowledge model, based on relationships between the one or more trial use case MW context parameters of the trial use case text and the plurality of MW infotypes present in the clinical trial knowledge model;
identifying a second set of MW infotypes out of the plurality of MW infotypes present in the clinical trial knowledge model by inferring the first set of MW infotypes with respect to the one or more recommendation rules of the clinical trial knowledge model;
identifying a third set of MW infotypes out of the plurality of MW infotypes present in the clinical trial knowledge model using one or more inclusion and exclusion dependency relationships obtained for the first set of MW infotypes and the second set of MW infotypes; and
combining the first set of MW infotypes, the second set of MW infotypes, and the third set of MW infotypes, to obtain the plurality of relevant MW infotypes.

12. The system as claimed in claim 8, wherein the one or more hardware processors (104) are configured to recommend the plurality of relevant MW concepts, using the one or more trial use case MW context parameters and one or more trial use case MW infotypes identified from the trial use case text, by:
identifying a first set of MW concepts out of the plurality of MW concepts present in the clinical trial knowledge model, based on (i) relationships between the plurality of relevant MW infotypes and the plurality of MW concepts present in the clinical trial knowledge model, and (ii) relationships between the one or more trial use case MW context parameters and the plurality of MW concepts present in the clinical trial knowledge model;
identifying a second set of MW concepts out of the plurality of MW concepts present in the clinical trial knowledge model by inferring the first set of MW concepts and the plurality of relevant MW infotypes with respect to the one or more recommendation rules of the clinical trial knowledge model;
identifying a third set of MW concepts out of the plurality of MW concepts present in the clinical trial knowledge model, using one or more inclusion and exclusion dependency relationships obtained for the first set of MW concepts and the second set of MW concepts; and
combining the first set of MW concepts, the second set of MW concepts, and the third set of MW concepts, to obtain the plurality of relevant MW concepts.

13. The system as claimed in claim 8, wherein the one or more hardware processors (104) are configured to generate the plurality of clinical trial documents for the trial use case text, using the clinical trial recommendation model and the clinical trial template model, by:
creating a clinical trial document for each MW template in the clinical trial template model, wherein each MW template comprises a plurality of MW sections and one or more MW sub-sections in each of the plurality of MW sections, wherein each MW section comprises a section name and a section description, and each sub-section comprises a sub-section name and a sub-section description;
matching the section name of each of the plurality of MW sections and the sub-section name of each of the one or more MW sub-sections, with the plurality of relevant MW infotypes and the plurality of relevant MW concepts using the one or more NLP techniques;
generating a MW concept description for each of the plurality of relevant MW concepts, using the plurality of relevant MW infotypes;
inserting the MW concept description for each of the plurality of MW sections and each of the one or more MW sub-sections whose section name, and sub-section name is matching with the plurality of relevant MW concepts;
inserting a MW infotype name for each of the plurality of MW sections and each of the one or more MW sub-sections whose section name, and sub-section name is matching with the plurality of relevant MW infotypes;
generating a section description for each of the plurality of MW sections and each of the one or more MW sub-sections whose section name, and sub-section name is not matching with the plurality of relevant MW concepts and the plurality of relevant MW infotypes, using the plurality of relevant MW infotypes; and
saving the clinical trial document created for each MW template, to obtain the plurality of clinical trial documents for the trial use case text, from the plurality of MW templates.

14. The system as claimed in claim 8, wherein the one or more hardware processors (104) are further configured to:
receive one or more recommendations from a user, based on the plurality of clinical trial documents generated for the trial use case text; and
update the clinical trial knowledge model with the one or more recommendations.

15. One or more non-transitory machine-readable information storage mediums comprising one or more instructions which when executed by one or more hardware processors cause:
receiving (i) one or more medical writing boilerplates associated with one or more disease types, (ii) one or more historical clinical trial documents, (iii) a plurality of clinical trial standard template documents defined for a drug regulatory authority, and (iv) a trial use case text to perform clinical trials for a drug ;
preprocessing (i) the one or more medical writing boilerplates associated with the one or more disease types, and (ii) the one or more historical clinical trial documents, using one or more natural language processing (NLP) techniques, to obtain (i) one or more pre-processed medical writing boilerplates associated with the one or more disease types, and (ii) one or more pre-processed historical clinical trial documents ;
executing one or more knowledge extraction patterns, on (i) the one or more pre-processed medical writing boilerplates associated with the one or more disease types, and (ii) the one or more pre-processed historical clinical trial documents, using a clinical trial knowledge meta model, to extract a clinical trial knowledge model, and wherein the clinical trial knowledge model comprises instances of (i) a plurality of medical writing (MW) concepts, (ii) a plurality of MW infotypes, (iii) a plurality of MW contexts, (iv) a plurality of MW groups, (v) one or more relations between each of the plurality of MW concepts, the plurality of MW infotypes, the plurality of MW contexts, and the plurality of MW groups, and (vi) one or more recommendation rules;
preprocessing the trial use case text, using the one or more natural language processing (NLP) techniques, the clinical trial knowledge model, and a domain dictionary, to identify one or more trial use case MW context parameters and one or more trial use case MW infotypes, and to create a clinical trial instance of the trial use case text ;
recommending (i) a plurality of relevant MW infotypes and (ii) a plurality of relevant MW concepts, using the one or more trial use case MW context parameters and the one or more trial use case MW infotypes identified from the trial use case text ;
generating a clinical trial recommendation model, by attaching the plurality of relevant MW infotypes, the plurality of relevant MW concepts and the one or more trial use case MW context parameters, to the clinical trial instance, using a clinical trial recommendation meta model ;
executing a template extraction pattern on the plurality of clinical trial standard template documents, using a clinical trial template meta model, to extract a clinical trial template model, wherein the clinical trial template model comprises a plurality of MW templates;
generating a plurality of clinical trial documents for the trial use case text, using the clinical trial recommendation model and the clinical trial template model;
receiving one or more recommendations from a user, based on the plurality of clinical trial documents generated for the trial use case text; and
updating the clinical trial knowledge model with the one or more recommendations.
